Europäisches Patentamt

European Patent Office

Office européen des brevets

(11) Veröffentlichungsnummer: **0 373 285**

**A1**

## EUROPÄISCHE PATENTANMELDUNG

(21) Anmeldenummer: **89105970.1**

(22) Anmeldetag: **05.04.89**

(51) Int. Cl.5: **A61B 6/00, A61B 6/06, G21K 1/04**

Geänderte Patentansprüche gemäss Regel 86 (2) EPÜ.

(30) Priorität: **16.12.88 US 285485**

(43) Veröffentlichungstag der Anmeldung:
**20.06.90 Patentblatt 90/25**

(84) Benannte Vertragsstaaten:
**BE DE FR GB**

(71) Anmelder: **Siemens Aktiengesellschaft**
**Wittelsbacherplatz 2**
**D-8000 München 2(DE)**

(72) Erfinder: **Hartwell, Gary**
**RT. No. 1 Box 411**
**Troy, Virginia 22974(US)**

(54) **Einblendvorrichtung für ein Röntgendiagnostikgerät.**

(57) Bei dem Röntgenstrahler eines Röntgendiagnostikgerätes weist ein Element (3.1) einer Einblendvorrichtung (3) an einer in Richtung zum Zentralstrahl zeigenden Seite einen von dieser Seite ausgehenden bogenförmigen Ausschnitt (3.2) auf. Durch die veränderbare Anordnung des bogenförmigen Ausschnittes (3.2) des Elementes (3.1) der Einblendvorrichtung (3) in einer Ebene senkrecht zum Nutzstrahlenbündel (4) kann dieses individuell auf ein bogenförmig verlaufendes Untersuchungsobjekt eingeblendet werden.

FIG 1

EP 0 373 285 A1

## Einblendvorrichtung für ein Röntgendiagnostikgerät

Die Erfindung betrifft ein Röntgendiagnostikgerät zur röntgenologischen Mammadiagnostik mit einer im Strahlengang der Röntgenröhre angeordneten Einblendvorrichtung mit einem verstellbaren Element, dessen eine Seite in Richtung zum Nutzstrahlenbündel angeordnet ist, zum Einblenden des von der Röntgenröhre abgestrahlten Nutzstrahlenbündels.

Bei bekannten Röntgendiagnostikgeräten wird die von der Röntgenröhre emittierte Röntgenstrahlung durch eine im Strahlengang der Röhre angeordnete Einblendvorrichtung auf das zu untersuchende Objekt eingeblendet. Die Einblendvorrichtung besteht aus plattenförmigen Elementen, die in einer Ebene senkrecht zum Zentralstrahl der Röntgenröhre verschiebbar angeordnet sind, so daß das Nutzstrahlenbündel im Bereich des Untersuchungsobjektes auf eine quadratische oder rechteckige Fläche eingeblendet werden kann.

Diese Einblendvorrichtung ist ungeeignet, das Nutzstrahlenbündel auf ein bogenförmig verlaufendes Untersuchungsobjekt, wie z.B. die weibliche Brust, einzublenden.

Aufgabe der Erfindung ist es daher, die Einblendvorrichtung eines Röntgendiagnostikgerätes derart auszuführen, daß das Nutzstrahlenbündel auf ein bogenförmig verlaufendes Untersuchungsobjekt eingeblendet werden kann.

Diese Aufgabe wird erfindungsgemäß dadurch gelöst, daß das Element einen von dieser Seite ausgehenden bogenförmigen Ausschnitt aufweist, so daß das Nutzstrahlenbündel an ein bogenförmig verlaufendes Untersuchungsobjekt angepaßt ist.

Vorteil dieser Ausführungsform ist, daß das Nutzstrahlenbündel optimal auf ein bogenförmig verlaufendes Untersuchungsobjekt, insbesondere in der Mammographie, eingeblendet werden kann, so daß die Strahlenbelastung für die zu untersuchende Person verringert und die Streustrahlung reduziert ist.

Vorteilhaft ist, wenn das Element im wesentlichen zylinderförmig und um seine Längsachse drehbar gelagert ist und wenn der bogenförmige Ausschnitt in bezug auf die Drehachse unsymmetrisch verläuft. Hierdurch kann das Nutzstrahlenbündel durch Drehen des zylinderförmigen Elementes individuell auf ein Untersuchungsobjekt eingeblendet werden.

Vorteilhaft ist, wenn der bogenförmige Ausschnitt in bezug auf eine Ebene senkrecht zur Drehachse und in Richtung des Zentralstrahls des Nutzstrahlenbündels symmetrisch verläuft. Hierdurch ist das Nutzstrahlenbündel symmetrisch zum Zentralstrahl einblendbar.

Eine weitere Ausgestaltung der Erfindung besteht darin, daß das Element plattenförmig ausgebildet und in einer Ebene senkrecht zum Zentralstrahl verschiebbar angeordnet ist, so daß das Nutzstrahlenbündel auf ein bogenförmig verlaufendes Untersuchungsobjekt individuell eingeblendet werden kann.

Vorteilhaft ist, wenn die Einblendvorrichtung an der dem Untersuchungsobjekt zugewandten Seite des Gehäuses des Röntgenstrahlers angeordnet und in einer Ebene senkrecht zum Zentralstrahl verschiebbar gelagert ist, da damit das Nutzstrahlenbündel auf ein zum Zentralstrahl dezentral angeordnetes Untersuchtungsobjekt eingeblendet werden kann.

Ausführungsbeispiele der Erfindung werden nachfolgend anhand der Figuren beschrieben. Dabei zeigt:

FIG 1 eine Einblendvorrichtung nach der Erfindung mit einem zylinderförmigen Element zum bogenförmigen Einblenden des Nutzstrahlenbündels und

FIG 2 ein plattenförmiges Element zum bogenförmigen Einblenden des Nutzstrahlenbündels.

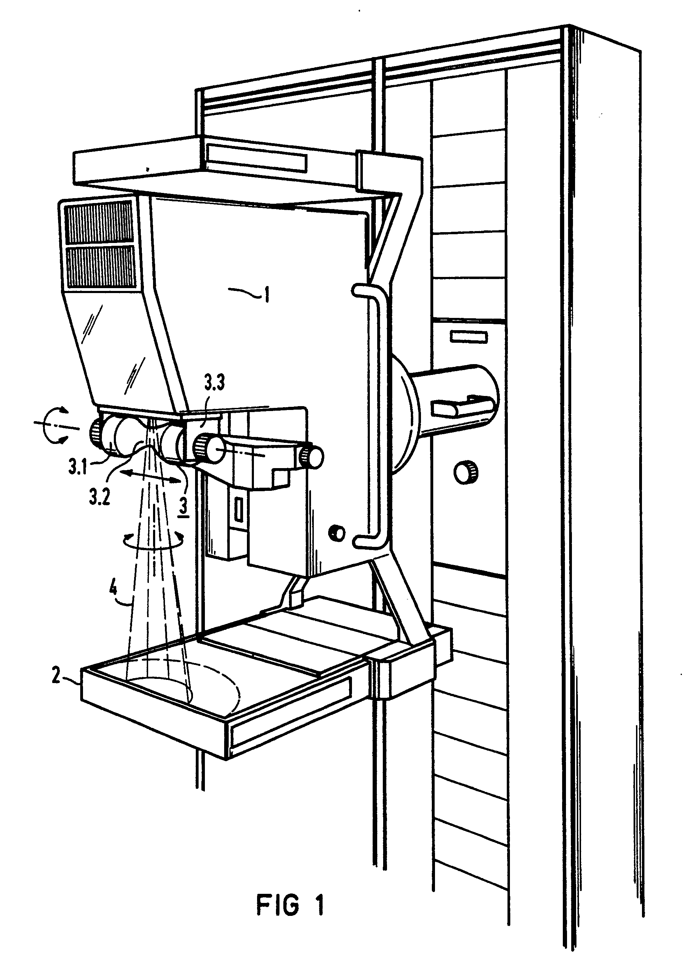

Die FIG 1 zeigt einen Röntgenstrahler 1 mit einer zum Nutzstrahlenbündel 4 der Röntgenröhre senkrecht angeordneten Untersuchungsebene 2. Auf der der Untersuchungsebene 2 zugewandten Seite des Röntgenstrahlers 1 ist eine Einblendvorrichtung 3 mit einem zylinderförmigen Element 3.1 zum Einblenden des Nutzstrahlenbündels 4 angeordnet. Das zylinderförmige Element 3.1 weist einen bogenförmigen Ausschnitt 3.2 auf, der in bezug zur Längsachse des zylinderförmigen Elementes 3.1 unsymmetrisch verläuft. Das zylinderförmige Element 3.1 ist um die Längsachse drehbar gelagert, so daß das Nutzstrahlenbündel 4 durch Drehen des zylinderförmigen Elementes 3.1 und bedingt durch den bogenförmigen Ausschnitt 3.2 auf jedes bogenförmig verlaufende Untersuchungsobjekt, wie z.B. die weibliche Brust, eingeblendet werden kann. Im Ausführungsbeispiel ist das zylindrische Element 3.1 zusätzlich in Richtung der Längsachse verschiebbar gelagert. Damit kann das Nutzstrahlenbündel 4 durch Verschieben des zylinderförmigen Elementes 3.1 in Richtung der Längsachse auch auf ein dezentral gelagertes Untersuchungsobjekt symmetrisch eingeblendet werden. Gleichwirkend ist selbstverständlich die entsprechende Verschiebung der gesamten Einblendvorrichtung 3 mit ihrer Halterung. Außerdem kann die Einblendvorrichtung 3 um den Zentralstrahl oder um eine Parallele dazu drehbar gelagert sein, um jeweiligen Aufnahmebedürfnissen gerecht zu werden. Durch die Anordnung des zylinderförmigen Elementes 3.1 im Nutzstrahlenbündel der Röntgenröhre wird von der die Strahlung begrenzenden Seite ausgehend ein halbtransparenter Übergangsbereich erhalten.

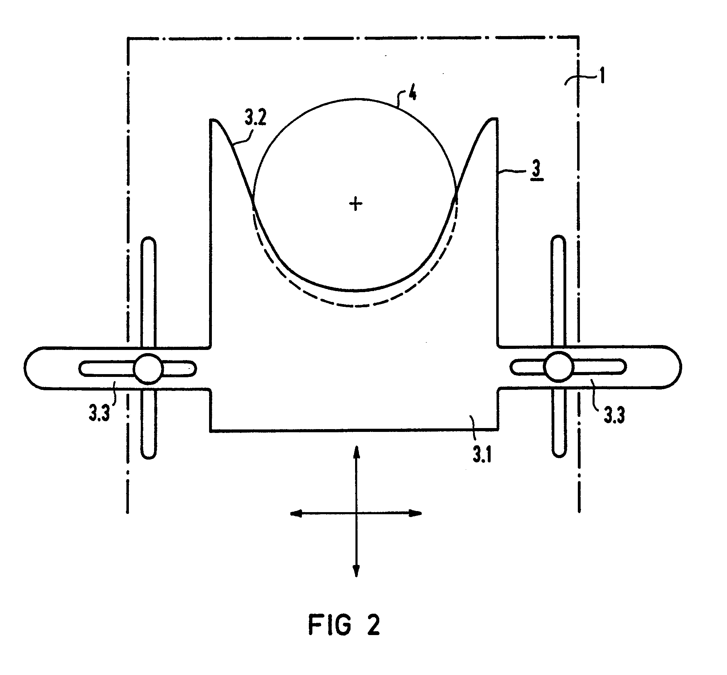

Ähnlich wirkend ist die plattenförmige Einblendvorrichtung 3 nach FIG 2. Das plattenförmige Element 3.1 ist durch die Befestigungselemente 3.3 auf der dem Untersuchungsobjekt zugewandten Seite des Röntgenstrahlers 1 verschiebbar gelagert. Das plattenförmige Element 3.1 weist auf der dem Nutzstrahlenbündel 4 der Röntgenröhre zugewandten Seite einen bogenförmigen Ausschnitt 3.2 auf. Durch Verschieben des plattenförmigen Elementes 3.1 im Nutzstrahlenbündel der Röntgenröhre ist dieses individuell auch auf dezentral gelagerte Untersuchungsobjekte bogenförmig einblendbar.

## Ansprüche

1. Röntgendiagnostikgerät zur röntgenologischen Mammadiagnostik mit einer im Strahlengang der Röntgenröhre angeordneten Einblendvorrichtung (3) mit einem verstellbaren Element (3.1), dessen eine Seite in Richtung zum Nutzstrahlenbündel (4) angeordnet ist, zum Einblenden des von der Röntgenröhre abgestrahlten Nutzstrahlenbündels (4), **dadurch gekennzeichnet**, daß das Element (3.1) einen von dieser Seite ausgehenden bogenförmigen Ausschnitt (3.2) aufweist, so daß das Nutzstrahlenbündel (4) an ein bogenförmig verlaufendes Untersuchungsobjekt angepaßt ist.

2. Röntgendiagnostikgerät nach Anspruch 1, **dadurch gekennzeichnet**, daß das Element (3.1) im wesentlichen zylinderförmig und um seine Längsachse drehbar gelagert ist und daß der bogenförmige Ausschnitt (3.2) in bezug auf die Drehachse unsymmetrisch verläuft.

3. Röntgendiagnostikgerät nach Anspruch 2, **dadurch gekennzeichnet**, daß der bogenförmige Ausschnitt (3.2) in bezug auf eine Ebene senkrecht zur Drehachse und in Richtung des Zentralstrahls des Nutzstrahlenbündels (4) symmetrisch verläuft.

4. Röntgendiagnostikgerät nach Anspruch 1, **dadurch gekennzeichnet**, daß das Element (3.1) plattenförmig ausgebildet und in einer Ebene senkrecht zum Zentralstrahl verschiebbar angeordnet ist, so daß das Nutzstrahlenbündel (4) auf ein bogenförmig verlaufendes Untersuchungsobjekt individuell eingeblendet werden kann.

5. Röntgendiagnostikgerät nach einem der Ansprüche 1 bis 4, **dadurch gekennzeichnet**, daß die Einblendvorrichtung (3) an der dem Untersuchungsobjekt zugewandten Seite des Gehäuses des Röntgenstrahlers (1) angeordnet und in einer Ebene senkrecht zum Zentralstrahl verschiebbar gelagert ist.

6. Röntgendiagnostikgerät nach einem der Ansprüche 1, 2, 3 und 5, **dadurch gekennzeichnet**, daß das zylindrische Element (3.1) in axialer Richtung verschiebbar gelagert ist.

7. Röntgendiagnostikgerät nach einem der Ansprüche 1 bis 6, **dadurch gekennzeichnet**, daß die Einblendvorrichtung (3) um den Zentralstrahl drehbar gelagert ist.

8. Röntgendiagnostikgerät nach einem der Ansprüche 1 bis 6, **dadurch gekennzeichnet**, daß die Einblendvorrichtung (3) um eine Parallele zum Zentralstrahl drehbar gelagert ist.

Geänderte Patentansprüche gemäss Regel 86(2) EPU.

1. Röntgendiagnostikgerät zur röntgenologischen Mammadiagnostik mit einer im Strahlengang der Röntgenröhre angeordneten Einblendvorrichtung (3) mit einem verstellbaren Element (3.1), das das Nutzstrahenbündel (4) mit einem bogenförmigen Ausschnitt einblendet, **dadurch gekennzeichnet**, daß das Element (3.1) im wesentlichen zylinderförmig und um seine Längsachse drehbar gelagert ist und daß der bogenförmige Ausschnitt (3.2) in bezug auf die Drehachse unsymmetrisch verläuft.

2. Röntgendiagnostikgerät nach Anspruch 1, **dadurch gekennzeichnet**, daß der bogenförmige Ausschnitt (3.2) in bezug auf eine Ebene senkrecht zur Drehachse und in Richtung des Zentralstrahls des Nutzstrahlenbündels (4) symmetrisch verläuft.

3. Röntgendiagnostikgerät nach Anspruch 1 oder 2, **dadurch gekennzeichnet**, daß das zylindrische Element (3.1) in axialer Richtung verschiebbar gelagert ist.

4. Röntgendiagnostikgerät nach einem der Anspruch 1 bis 3, **dadurch gekennzeichnet**, daß die Einblendvorrichtung (3) an der dem Untersuchungsobjekt zugewandten Seite des Gehäuses des Röntgenstrahlers (1) angeordnet und in einer Ebene senkrecht zum Zentralstrahl verstellbar gelagert ist.

5. Röntgendiagnostikgerät nach einem der Ansprüche 1 bis 4, **dadurch gekennzeichnet**, daß die Einblendvorrichtung (3) um den Zentralstrahl drehbar gelagert ist.

FIG 1

FIG 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | FR-A-2 083 086 (SIEMENS AG) <br> * Insgesamt * <br> --- | 1,3,4 | A 61 B 6/00 <br> A 61 B 6/06 <br> G 21 K 1/04 |
| X | EP-A-0 178 728 (PHILIPS GLOEILAMPENFABRIKEN) <br> * Seite 3, Zeile 10 - Seite 4, Zeile 25; Seite 6, Zeile 22 - Seite 7, Zeile 11; Figuren 1,5,6 * <br> --- | 1,7,8 | |
| A | EP-A-0 059 382 (GENERAL ELECTRIC CO.) <br> * Zusammenfassung; Seite 8, Zeilen 20-28; Figur 1 * <br> --- | 1,2 | |
| A | CA-A-1 147 069 (P. CHARRIER) <br> * Seite 1, Zeilen 1-10; Seite 3, Zeile 28 - Seite 5, Zeile 14; Figuren 1-3 * <br> ------ | 1,2 | |

RECHERCHIERTE
SACHGEBIETE (Int. Cl.5)

A 61 B
G 21 K

**Der vorliegende Recherchenbericht wurde für alle Patentansprüche erstellt**

| Recherchenort | Abschlußdatum der Recherche | Prüfer |
|---|---|---|
| DEN HAAG | 25-09-1989 | FERRIGNO, A. |